# EUROPEAN PATENT SPECIFICATION

(11) **EP 2 925 881 B1**
(45) Date of publication and mention of the grant of the patent: **15.11.2017**
(21) Application number: 13857774.7
(22) Date of filing: 28.11.2013
(51) Int. Cl.: C12Q 1/48, C12Q 1/68, G01N 33/574

(54) **N-MYRISTOYLTRANSFERASE 2 OVEREXPRESSION IN PERIPHERAL BLOOD AND PERIPHERAL BLOOD MONONUCLEAR CELLS IS A MARKER FOR COLORECTAL CANCER**
2 N-MYRISTOYLTRANSFERASE-2-ÜBEREXPRESSION IN PERIPHERBLUT UND PERIPHEREN MONONUKLEAREN BLUTZELLEN ALS MARKER FÜR KOLOREKTALKARZINOME
LA SUREXPRESSION DE N-MYRISTOYLTRANSFÉRASE 2 DANS LE SANG PÉRIPHÉRIQUE ET DES CELLULES MONONUCLÉÉES DU SANG PÉRIPHÉRIQUE EST UN MARQUEUR POUR LE CANCER COLORECTAL

(30) Priority: 28.11.2012 US 201261730694 P
(43) Date of publication of application: 07.10.2015
(73) Proprietor: Shrivastav, Anuraag, Winnipeg, Manitoba R3B 2E9 (CA)
(72) Inventor: Shrivastav, Anuraag, Winnipeg, Manitoba R3B 2E9 (CA)
(74) Representative: Franke, Dirk
(86) International application number: PCT/CA2013/050913
(87) International publication number: WO 2014/082178

(56) References cited:
- WO-A1-2007/036025
- TAKAMUNE N ET AL: "Novel strategy for anti-HIV-1 action: selective cytotoxic effect of N-myristoyltransferase inhibitor on HIV-1-infected cells", FEBS LETTERS, ELSEVIER, AMSTERDAM, NL, vol. 527, no. 1-3, 11 September 2002 (2002-09-11), pages 138-142, XP004380561, ISSN: 0014-5793, DOI: 10.1016/S0014-5793(02)03199-X
- SELVAKUMAR, P. ET AL.: 'N-Myristolvlhansferase 2 expression in human colon cancer: cross-talk between the calpain and caspase system' FEBS LETTERS vol. 580, no. 8, April 2006, pages 2021 - 6, XP028030344

## Description

### PRIOR APPLICATION INFORMATION

The instant application claims the benefit of US Provisional Patent Application 61/730,694, filed November 28, 2012.

### BACKGROUND OF THE INVENTION

Cancer is the leading cause of death in Canada. Colorectal cancer (CRC), the second most fatal, has a 90% survival if treated at an early stage (CancerCare Manitoba, 2007; Population screening for colorectal cancer, 2006). Yet, every year over 600,000 people around the world die of CRC (Canadian Cancer Society, 2010). CRC arises from pre-malignant adenomatous polyps, which may take several years to develop into cancer. Genetics may also play a role, as 30% of CRC cases are familial (CancerCare Manitoba, 2007; Population screening for colorectal cancer, 2006). However, not all polyps develop into CRC and the vast majority of CRC cases are not familial. These statistics highlight the urgent need for reliable screening methods for early detection of CRC.

### Current CRC Screening Strategies

Screening is the means of identifying individuals at risk to a disease prior to the development of symptoms. The most common screening tests for CRC include Fecal Occult Blood Testing (FOBT), Sigmoidoscopy and Colonoscopy (Screening, 2001) (Winawer, et al., 2003). The compliance rate of these tests for screening is limited due to low sensitivity or invasive nature (Moayyedi, 2007; Nicholson, et al., 2005). For instance, though FOBT is cost effective and relatively safe, the false positive rates are high and factors such as medication or diet may skew results. For FOBT screening, patients must collect a stool sample at home and bring it to a laboratory for analysis. The unpleasant nature of fecal sample collection has led to low patient compliance. In Manitoba, colon cancer screening has been largely unsuccessful as 85% of targeted patients chose not to undergo the process. Sigmoidoscopy and colonoscopy are both expensive and invasive, and the results and risks of the procedure depend on the expertise of the attending endoscopist (Baxter and Rabeneck, 2009; Singh et al. 2010; Singh et al. 2009).

The widely available biomarker Carcinoembryonic Antigen (CEA) has limited sensitivity and specificity (Duffy, et al., 2003; Ouyang, et al., 2005). However, blood tests are likely to be more readily acceptable than stool or endoscopic tests. Cost effective blood tests may identify patients at high risk for CRC and improve patient compliance for more intensive and invasive diagnostic procedures.

### N-myristoylation

N-myristoylation is the covalent attachment of a 14-carbon saturated fatty acid chain to the N-terminal glycine residue of a protein that is catalyzed by the enzyme N-myristoylatransferase (NMT). Myristoylation of proteins has been observed across diverse taxa, including that of mammals, plants, viruses and fungi (Farazai, et al., 2001). In lower eukaryotes a single gene codes for NMT. Higher eukaryotes such as humans have two genes (Giang and Cravatt, 1998).

N-myristoylation is an irreversible co-translational protein modification (Towler, et al., 1987). Some recent reports have suggested exceptions to this rule with evidence of post-translational myristoylation. For instance, the pro-apoptotic protein BID is cleaved by Caspase 8 prior to apoptosis to reveal a myristoylation motif. Another protein that is post-translationally myristoylated is P21-activated protein kinase, which is involved in maintaining the cytoskeleton (Zha, et al., 2000; Vilar, et al., 2006). Proteins involved in signal cascades, cellular transformation and oncogenesis are often myristoylated. These include the catalytic subunit of cAMP-dependent protein kinase (Carr, et al., 1982), the β-subunit of Calcineurin (Aitken, et al., 1982), the α-subunit of several G-proteins (Schultz, et al, 1987), the cellular transforming forms of pp^{60-src} (Schultz, et al., 1985), several tyrosine kinases and proteins important for assembly, maturation and infectivity of mature virus particles, such as murine leukemia virus Pr65gag precursor (Rein, et al., 1986) and poliovirus VPO polypeptide precursor (Marc, et al., 1989).

Regarding viral infections, it was found that NMT 1 and NMT2 mRNA as well as NMT1 and NMT2 activity are decreased in the course of an HIV-1 infection in the human T-cell line CEM. Furthermore, inhibitors of NMT were found to exhibit a cytotoxic effect on HIV-1 infected CEM cells (Takamune et al., 2002).

### NMT Overexpression in CRC

Eukaryotic NMT is a member of the GCN5-related N-acetyltransferase (GNAT) superfamily of proteins (Resh, 1999) (Boutin, 1997) (Farazai, et al., 2001). N-acetyltransferase uses acetyl coenzyme A (CoA) to transfer an acetyl group from the donor to the primary amine of the acceptor. Two genes encode NMT in higher eukaryotes such as bovine, human and plants. The second genetically distinct NMT 2 cDNA (NMT-2) has been cloned from a human liver library. The respective mouse homologues for the two human NMTs have also been cloned (Giang DK et al., 1998, J Biol Chem.;273:6595-8). NMT2 protein is a product of different gene (*NMT2* gene) than *NMT1.*

Elevated activity of NMT has been reported in colonic tumour tissue as compared to tissue adjacent to the tumour and tissue from control patients (Magnuson, et al., 1995). Recent studies have also shown that NMT1 expression in colonic tumours is higher during the early stages of colon cancer, and is also high in polyps (Selvakumar, et al., 2006). However, in previous studies, measurements of NMT activity and expression in tumour tissues by IHC were most likely depicting total NMT expression instead of just NMT1 (King & Sharma, 1991).

### NMT overexpression

NMT overexpression in colonic tumours is not well understood. However, it is consistent with the increased demands for myristoylation of oncoproteins in response to rapid cell division during tumorigenesis. A link between colorectal cancer (CRC) and the immune system can be established through their respective demands of NMT. NMT activity and NMT1 expression has been found to be essential for the proper development of monocytic lineage, and therefore may be involved in the differentiation of other leukocytes (Shrivastav, et al., 2008). Shrivastav et al. (2007) established strong positive NMT1 immunostaining in CRC peripheral blood of CRC patients (n=18). The immuno-staining was performed using polyclonal antibody that was raised against full length NMT1 protein. Since NMT1 share about 77% amino acid sequence homology with NMT2, therefore, this NMT1 polyclonal antibody might have recognized both NMT1 and NMT2 proteins in the peripheral blood samples or else due to the activation of immune response that leads to increased NMT1 expression due to infection and/or cancer.

### SUMMARY OF THE INVENTION

Previously, it was reported NMT1 protein is overexpressed in PBMC of colorectal cancer patients due to positive immunostaining using polyclonal antibody raised against full length NMT1 protein.

The inventors' current study of the expression pattern of NMT1 and NMT2 isoforms (products of two different genes) in PBMC of CRC patients using antibodies specific to NMT1 or NMT2 showed that NMT2 protein and not NMT1 protein is overexpressed in the PBMC of CRC patients. NMT2 protein is the product of a different gene than NMT1. Upon further isolation of T-cells from PBMC the inventors clearly established that NMT2, not NMT1, is overexpressed in T cells of CRC patients. The inventors used validated polyclonal prestige antibodies from Sigma (Canada), which are specific to NMT1 or NMT2. Unique recognition of NMT1 and NMT2 polyclonal antibodies enabled the inventors to delineate that NMT2 is overexpressed in T cells of CRC patients. Due to lack of NMT isoform specific antibody in earlier study the inventors in fact were measuring total NMT (NMT1 and NMT2) expression by immunohistochemistry rather than just NMT1.

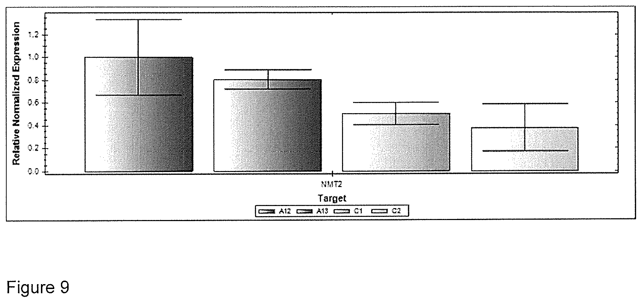

Moreover in a recent study the inventors also observed increase in NMT1 expression when resting CD4+ T cells were activated by T cell ligation using ant-CD3/CD46 antibody. This adds complexity in measuring NMT1 in CRC patients as their CD4+ T cells are activated because of immune response due to several reasons including infection and or cancer. (Figure 9).

According to an aspect of the invention, there is provided a method of identifying a candidate for further colorectal cancer (CRC) screening comprising: measuring N-myristoylatransferase 2 (NMT2) levels in a sample from a patient at risk of developing CRC, suspected of having CRC or having CRC, wherein NMT2 levels above a threshold level indicates that the patient is a candidate for further CRC screening.

The further CRC screening method may be selected from the group consisting of: sigmoidoscopy and colonoscopy.

The NMT2 levels may be measured by using a primer or probe comprising nucleic acid sequence unique to NMT2 or using an antibody specific for NMT2.

The patient may be a human.

The patient may have a familial history of colorectal cancer or is older than 55.

The sample may be selected from the group consisting of: whole blood, peripheral blood mononuclear cells, T-cells and/or its subpopulations including CD8+ cells. Alternatively, the sample may be a CD4 cell sample, that is, a sample that either includes CD4 cells or is enriched for CD4 cells, that is, has a greater than normal proportion of CD4 cells.

According to another aspect of the invention, there is provided a method of measuring N-myristoylatransferase 2 (NMT2) levels in a sample from a patient comprising: measuring N-myristoylatransferase 2 (NMT2) levels in a sample from a patient by using a measuring reagent which is specific to NMT2 compared to NMT1, and determining if NMT2 levels are above a threshold level.

### BRIEF DESCRIPTION OF THE DRAWINGS

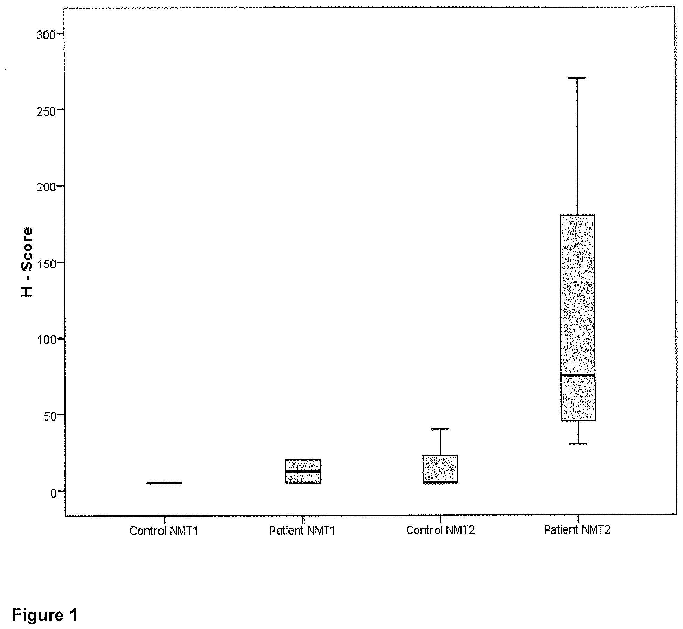

FIGURE 1: NMT1 and NMT2 expression profile in CD4+ T cells from control (n=4) and CRC patients (n=4). NMT1 and NMT2 appear to be higher in CRC patients. NMT1 expression has high overlap between patients is not significant. Whereas NMT2 expression shows some overlap. This is likely due the lowest value from patient ID023 with an H-score of 30 and the highest H-score value of 40 from control group (CS2).

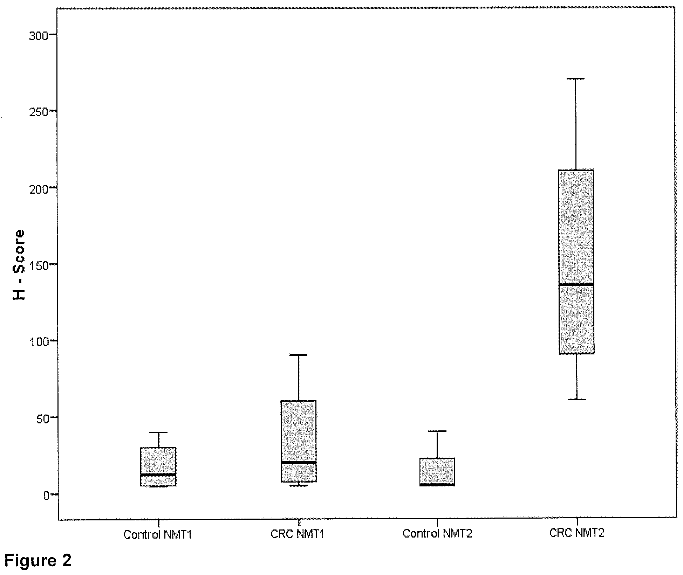

FIGURE 2: NMT1 and NMT2 expression profile in CD8+ T cells from control and CRC patients. NMT1 and NMT2 appear to be higher in CRC patients but there is a fair amount of overlap. T test comparing NMT1 in CD8+ T cells was found to be insignificant. NMT2 overexpression was found to be significant by T test.

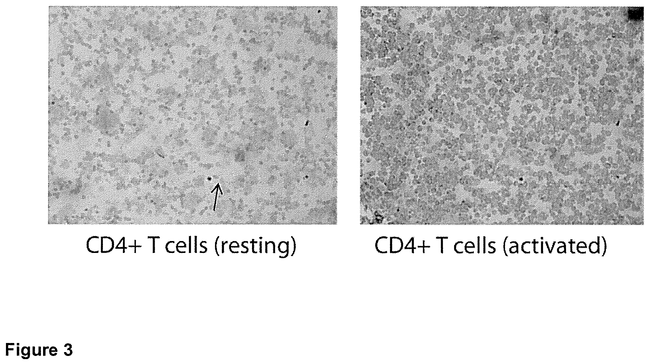

FIGURE 3: Representative IHC picture of CD4+ T cells stained for NMT1 using DAB chromagen (brown stain) at 40X. a) is from group 1 (patient CS1) and b) is from group 2 (ID018). CD4+ T cells from control patients (arrow) show virtually no staining for NMT1. CRC patients show lower levels of NMT1 and in fewer cells (arrow).

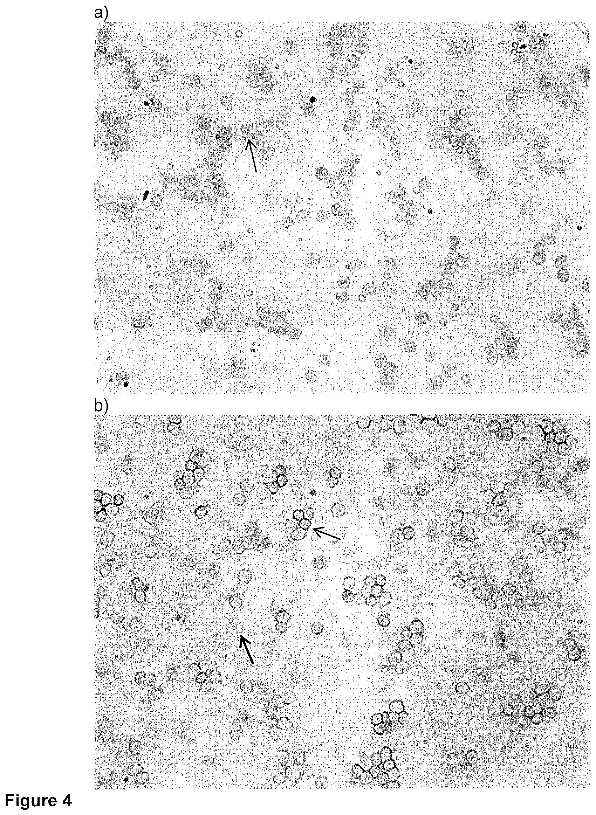

FIGURE 4: Representative IHC picture of CD4+ T cells stained for NMT2 using DAB chromagen (brown stain) at 40X. a) is from group 1 (patient CS1) and b) is from group 2 (ID018). CD4+ T cells from the control group show virtually no stain. CD4+ T cells from CRC patients show strong positive staining for NMT2 (arrow). It can also be observed that NMT2 is localised in the cytoplasm of T cells. However, not all cells appear to exhibit overexpression (fat arrow).

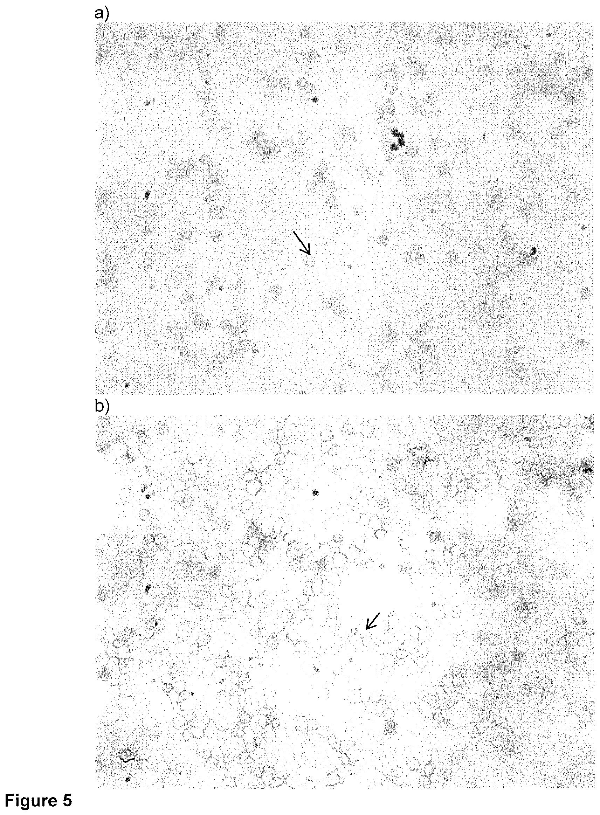

FIGURE 5: Representative IHC picture of CD8+ T cells stained for NMT1 using DAB chromagen (brown stain) at 40X. a) is from control group (CS1 and b) is from a CRC patient (ID018). Cells from control patients show virtually no staining (arrow). The spherical red dots are magnetic beads used in T cell isolation. Most of the cells from CRC patients have stained moderate positive for NMT1 (arrow).

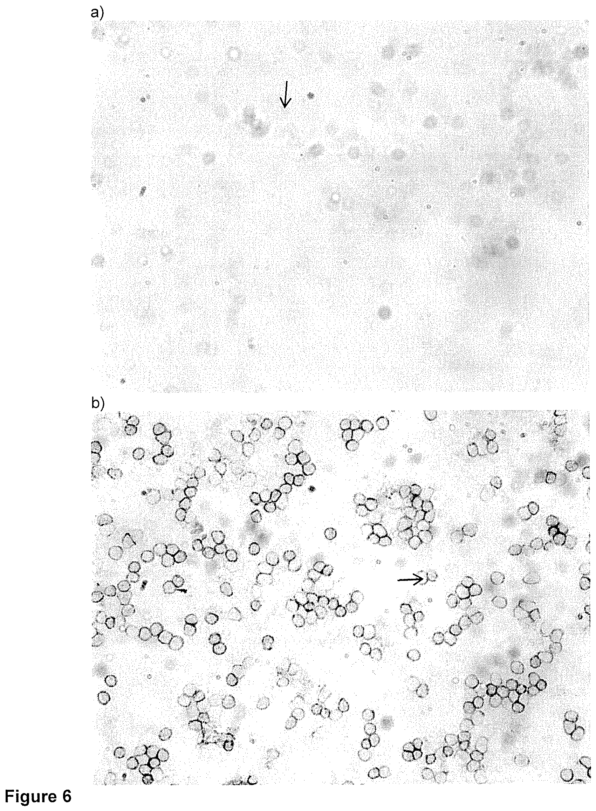

FIGURE 6: Representative IHC picture of CD8+ T cells stained for NMT2 using DAB chromagen (brown stain) at 40X. a) is from control group (CS1) and b) is from a CRC patient (ID017). Cells from control patients show virtually no staining (arrow). Most of the cells from CRC patients show strong positive staining for NMT2 (arrow).

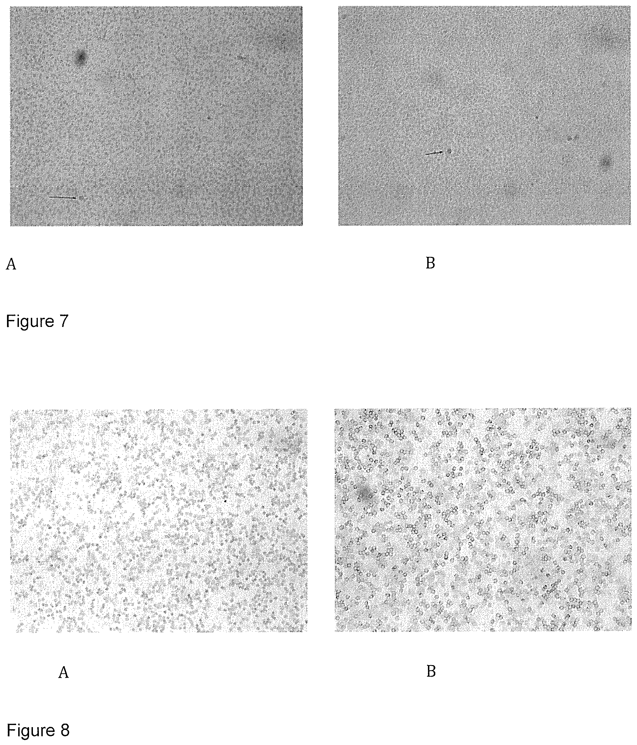

FIGURE 7: NMT2 expression in peripheral blood smears: Blood smear were made on glass slide and immunohistochemical analysis was performed as described in the material and method section. A) NMT2 expression in the peripheral blood cells. Lymphocytes from healthy subject (indicated by an arrow) display weak to negative staining for NMT2 whereas, B) NMT2 expression is high as determined by intensity of staining by anti-NMT2 antibody in the peripheral blood cells (lymphocyte as indicated by an arrow) from colorectal cancer patient.
FIGURE 8: NMT2 expression in peripheral blood mononuclear cells (PBMC): PBMC were separated from whole blood by density gradient centrifugation using ficoll-hypaque. Cytospin slides of PBMC were prepared and immunohistochemical analysis was performed as described in the material and method section. A) NMT2 expression in the peripheral PBMC from healthy subject display weak to negative staining for NMT2 whereas, B) NMT2 expression is high as determined by strong intensity of staining by anti-NMT2 antibody in the PBMC from colorectal cancer patient.
FIGURE 9. N-myristoyltransferase-2 (NMT2) gene expression profile in CRC patients and healthy subjects. NMT2 gene expression in the peripheral blood mononuclear cells was determined in CRC patients (A12 and A13) and healthy subjects (C1 and C2) by quantitative real time polymerase chain reaction (qRT-PCR) using validated PCR Prime primers from BioRad. These primers were specific for NMT2. The expression of NMT2 gene in CRC patients is twice as compared to healthy subjects.

### BRIEF DESCRIPTION OF THE TABLES

TABLE 1: Two tailed T-test results analysis from comparing NMT1 and NMT2 expression levels in patient and control groups.
TABLE 2: NMT1 expression profile of patients in CD4+ T cells and CD8+ T cells.
TABLE 3: NMT2 expression profile of patients in CD4+ T cells and CD8+ T cells.
TABLE 4: Medical backgrounds of participants.

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

Earlier reports revealed that NMT1 overexpression is most prominent in Peripheral Blood Mononuclear Cells (PBMC). In control subjects, NMT1 expression in PBMC and polymorphonuclear cells PMN ranged from negative to rare-weak positivity, with no more than 20% of cells ever staining positive. In contrast, CRC patients showed strong NMT1 staining in PBMC and PMNs. However in another report, it was suggested that expression of NMT1 is dependent on neutrophil activation state Shrivastav et al., 2010 Vet Res. 2010;41:9).. Also, the inventors reported earlier that NMT1 is the principal enzyme during embryogenesis and two isoforms are not redundant in function (Yang et al., 2005, J Biol Chem. 2005;280:18990-5).. It is possible that NMT1 is involved in normal functioning of blood cells, however, NMT2 overexpression is signature of colorectal oncogenic processes. It is quite possible that T cells overexpressing NMT2 might represent a host of antigenic specificities, and those against CRC. Therefore, NMT2 overexpression exclusively due to CRC-driven clonal expansion is one of the favoured scenarios. Whether NMT2 overexpression can also be due to constitutive overexpression (i.e., prior to the onset of disease) in individuals who are at high risk of developing CRC is another possibility.

Accordingly, the biological basis for NMT2 overexpression in the PBMC of CRC patients has to be better characterized to utilize NMT2 as a marker for early detection of CRC.

As discussed below, described herein is the Identification of the NMT isozyme, i.e. NMT2, overexpressed in PBMCs of CRC patients and the cell types overexpressing NMT2 in the PBMCs of CRC patients.

According to an aspect of the invention, there is provided a method of identifying a candidate for further colorectal cancer (CRC) screening comprising:
measuring N-myristoylatransferase 2 (NMT2) levels in a sample from a patient at risk of developing colorectal cancer, suspected of having colorectal cancer or having colorectal cancer, wherein NMT2 levels above a threshold level indicates that the patient is a candidate for further CRC screening.

According to another aspect of the invention, there is provided a method of measuring N-myristoylatransferase 2 (NMT2) levels in a sample from a patient comprising:
measuring N-myristoylatransferase 2 (NMT2) levels in a sample from a patient by using a measuring reagent which is directed to unique or specific regions of NMT2 compared to NMT1, and
determining if NMT2 levels are above a threshold level.

As discussed herein, the measuring reagent may be a primer, probe or antibody specific for NMT2.

According to an aspect of the invention, there is provided a method of determining if a patient is a candidate for further colorectal cancer (CRC) screening comprising:
measuring N-myristoylatransferase 2 (NMT2) levels in a sample from a patient at risk of developing colorectal cancer, wherein NMT2 levels above a threshold level indicates that the patient is a candidate for further CRC screening or diagnosis.

According to an aspect of the invention, there is provided a method of identifying a candidate for further colorectal cancer (CRC) screening comprising:
providing a sample from a patient at risk of developing colorectal cancer, and
measuring N-myristoylatransferase 2 (NMT2) levels in said sample, wherein NMT2 levels above a threshold level indicates that the patient is a candidate for further CRC screening.

According to an aspect of the invention, there is provided a method of determining if a patient is a candidate for further colorectal cancer (CRC) screening comprising:
providing a sample from a patient at risk of developing colorectal cancer; and
measuring N-myristoylatransferase 2 (NMT2) levels in said sample, wherein NMT2 levels above a threshold level indicates that the patient is a candidate for further CRC screening.

Preferably, in some embodiments, only NMT2 levels are measured. In some other embodiments, NMT2 levels are measured in isolation from or in the absence of NMT1 levels.

As will be appreciated by one of skill in the art, further colorectal cancer screening comprises any suitable screening method known in the art, for example but by no means limited to sigmoidoscopy and colonoscopy.

As will be appreciated by one of skill in the art, the NMT2 levels may be measured using any suitable means known in the art which will distinguish between NMT2 expression and NMT1 expression. For example, as discussed above, the sequences of both NMT1 and NMT2 are known and accordingly NMT2 levels may be detected for example by nucleic acid amplification based methods, by probes or by antibodies.

For example, a primer or probe specific to NMT2 may be used to detect NMT2 transcripts, for example by hybridization and/or amplification. Alternatively, antibodies, for example, a monoclonal antibody or a preparation of polyclonal antibodies raised against one or more specific regions of NMT2 amino acid sequence may be used to detect NMT2 levels. Specifically, a primer or probe or antibody "specific" for NMT2 is one that does not cross-react with or otherwise detect NMT1. Such reagents are well known in the art.

In some embodiments of the invention, NMT2 expression is measured with the proviso that no NMT1 levels are measured simultaneously.

As will be appreciated by one of skill in the art, the above examples are by no means exhaustive and other suitable methods for measuring NMT2 levels, for example, measuring NMT2 transcription levels or NMT2 expression levels, will be readily apparent to one of skill in the art.

Preferably the patient or individual is a human.

Preferably the patient or individual is an individual considered to be at risk of developing colorectal cancer, suspected of having colorectal cancer or having colorectal cancer. Such risk factors are well known to those of skill in the art. For example, the patient or individual may have a familial history of colorectal cancer or may be older than 55 or may have a history of polyp development.

The sample may be any suitable sample from which NMT2 activity can be measured. As will be appreciated by one of skill in the art, the "threshold" level referred to above will of course depend on the method of measuring NMT2 and will also depend on the sample used in this measurement. Such a threshold may be determined by comparison with a known negative control and/or a known positive control. Examples of suitable samples include but are by no means limited to whole blood, peripheral blood monocyte cells, T-cells and/or CD8+ cells.

Alternatively, the sample may be a CD4 cell sample, that is, a sample that either includes CD4 cells or is enriched for CD4 cells, that is, has a greater than normal proportion of CD4 cells.

Previous studies revealed that NMT1 was overexpressed in peripheral blood of CRC patients. Earlier study (Shrivastav et al 2007) narrowed down the exclusive NMT overexpression in CRC patients to PBMC. Since over 60% of PBMC are T cells, the inventor concluded that NMT2 overexpression exclusive to CRC is from a T cell subpopulation. The two largest subpopulations of T cells, the CD4+ T cells (Helper T cells) and CD8+ T cells (Cytotoxic T cells) were analysed for the expression of the two NMT isoforms, NMT1 and NMT2. Blood samples from control subjects (n=4) and CRC patients (n=4) were used for comparative analysis. 3 patients who did not suffer from CRC but showed elevated levels of NMT were also studied but analysed separately.

Earlier it was reported that NMT1 is overexpressed in PBMC of CRC patients (Shrivastav, et al., 2007). However, the polyclonal antibody used for IHC study was raised against full length NMT1. The NMT isoforms NMT1 and NMT2 share a 77% amino acid sequence homology. Therefore there is an ambiguity about which isozyme is being overexpressed in the peripheral blood of CRC patients. Therefore, the expression of NMT isoforms in T cell subpopulation was explored and it was observed that both NMT1 and NMT2 show altered expression in CD4+ T cells and CD8+ T cell subpopulations in CRC patients (Table 2 and 3).

NMT1 expression compared to NMT2 is much lower in CRC patients (Figures 1 and 2). Due to variation in NMT1 expression, it could not be established as being overexpressed. However, NMT2 isozyme expression was found to be significantly higher in CD4+ T cells and CD8+ T cells of CRC patients (Table 2).

NMT2 expression in CD4+ T cells of CRC patients was also high but there is a wide range of expression (Table 2). The variation is primarily from patient ID024 whose CD4+ T cell NMT2 expression is unusually low (H score - 30). ID024 has Stage 4 CRC with no polyps. There could be a link between presence of polyps and NMT2 expression in CD4+ T cells. ID008 does not have cancer, but has polyps and a strong family history, and subsequently the CD4+ T cells show a high NMT2 H score of 180 (Table 3). However, other CRC patients do not appear to have polyps but continue to overexpress NMT2.

Myristoylated proteins are involved in a range of functions, from cell division to apoptosis, but most are not well understood. Shrivastav et al. (2007) found NMT to localize in the nucleus of Bone Marrow Cells (BMC) from CRC patients and rats with colonic tumours. In control groups, NMT was cytoplasmic. Exploring the roles of NMT isoforms in CRC, Ducker et. al. (2005) examined the effects of silencing NMT1 and NMT2 using siRNA in CRC cells. Silencing NMT2 induced cell death in tumor cells 2.5 fold more as compared to silencing NMT1 (2005) that imply NMT2 is a better therapeutic target. It has also been observed that cancer causes hematopoietic stem cells to undergo apoptotic events (Deckers, et al., 1973).

A possible cause of NMT2 overexpression can be due to T cell differentiation process through the thymus. During T cell differentiation, there is a double positive stage (CD4+/CD8+ double positive T cells) where cells are tested for self-incompatibility. During this time, either CD4 or CD8 expression ceases in cells. Cells that fail the self-incompatibility test, around 96-98% of double positive T cells, undergo apoptosis (Orkin and Zon, 2008). CD8+ T cells, also called Cytotoxic T cells, function in tumour suppression by inducing apoptosis in cancer cells. If NMT2 suppresses apoptosis, then its overexpression might be a sign that defective CD8+ T cells can survive the apoptosis attempts induced by the thymus. This line of reasoning may also incorporate the putative role of nuclear localisation of NMT in bone marrow cells leads to NMT2 overexpression in T cells either directly or indirectly. This hypothesis suggests why many CD8+ T cells do not show NMT2 overexpression and possibly cells that do not overexpress NMT2 are not defective.

### NMT2 overexpression due to clonal expansion in response to CRC.

Naïve T cells are induced by CRC to overexpress NMT2 as they mature. Some of these mature T cells differentiate into memory T cells that retain high NMT2 expression levels. Clonal expansion from these memory T cells leads to production of mature cells which continue to overexpress NMT2. This hypothesis suggests that NMT2 overexpression may never subside in patients even if they have been treated for CRC.

A simpler explanation to NMT2 overexpression may involve the cell mediated immune response to CRC. In cell mediated anti-cancer response T cells mature in response to antigens from tumour. These mature T cells undergo clonal expansion (multiply) to suppress the tumour. Some clones from this clonal expansion become memory T cells that lie dormant until the antigen is encountered again. Upon re-exposure, these memory T cells can mount a faster response. Memory T cells can be either CD4+ or CD8+. Accordingly T cells that encountered CRC antigen(s) underwent transformation which resulted in NMT2 overexpression and clonally expanded in due course. The memory T cells formed in this response retained NMT2 overexpression. Thus, NMT2 overexpression persists after CRC has been resected (Table 4). According to this theory NMT2 overexpression has no bearing on the ability of a T cell to respond to CRC.

NMT2 in CD8+ T cells was found to be uniquely overexpressed in CRC patients. NMT2 expression in CD4+ T cells was comparable to that in CD8+ T cells but showed a wide range of expression and could not be established as statistically significant, but remains of interest. NMT1 expression was much lower than that of NMT2 in both T cell subtypes. NMT1 expression was highly variable in CRC patients and its overexpression could not be established as statistically significant varied expression could be due to activation of T cells because of infection and/or cancer. NMT2 in CD8+ T cells is clearly a screening tool for CRC.

Figure 7 demonstrates NMT2 expression in peripheral blood smears. Blood smears were made on glass slide and immunohistochemical analysis was performed. Shown in panel (A) is NMT2 expression in the peripheral blood cells. Lymphocytes from healthy subject (indicated by an arrow) display weak to negative staining for NMT2 whereas, as shown in panel (B), NMT2 expression is high as determined by intensity of staining by anti-NMT2 antibody in the peripheral blood cells (lymphocyte as indicated by an arrow) from colorectal cancer patient.

Figure 8 shows NMT2 expression in peripheral blood mononuclear cells (PBMC): PBMC were separated from whole blood by density gradient centrifugation using ficoll-hypaque. Cytospin slides of PBMC were prepared and immunohistochemical analysis was performed as described in the material and method section. Panel (A) shows NMT2 expression in the peripheral PBMC from healthy subject display weak to negative staining for NMT2 whereas, shown in panel (B), NMT2 expression is high as determined by strong intensity of staining by anti-NMT2 antibody in the PBMC from colorectal cancer patient.

Figure 9 shows NMT2 gene expression profile in CRC patients and healthy subjects. NMT2 gene expression in the peripheral blood mononuclear cells was determined in CRC patients (A12 and A13) and healthy subjects (C1 and C2) by quantitative real time polymerase chain reaction (qRT-PCR) using validated PCR Prime primers from BioRad. These primers were specific for NMT2. As can be seen, the expression of NMT2 gene in CRC patients is twice as compared to healthy subjects.

### NMT isoform distribution

In control subjects NMT2 appears to be expressed more than NMT1 in CD4+ T cells (Figure 1). NMT1 is virtually undetectable in CD4+T cells from control subjects. NMT isoform expression in CD8+ T cells was comparable in control subjects (Figure 2). NMT isoforms, when expressed at detectable levels were localised in the cytoplasm (Figures 3 and 4).

### Group 3 patients - High NMT expression and no CRC

Three subjects initially recruited as control were found to overexpress NMT (Table 4). ID002 has had a previous diagnosis of CRC and the tumour was resected. ID008 has a strong family history of CRC. ID013 was found to have a diverticulum, but family history is unknown. Due to their uncertain position as either controls or patients, or lack of histories, their results were not used in calculations.

### NMT isoform expression in CRC patients

In CRC patients, NMT2 expression is roughly 3 times higher than that of NMT1 in both T cell subtypes. On average, NMT1 expression was almost twice that from the control groups (Figure 1). However, NMT1 expression in CRC patients varies greatly and statistically cannot be proven as an overexpression (Table 1).

NMT2 is overexpressed in both T cell subtypes. Compared to the control group, NMT2 expression is roughly 5 times higher than controls (Figure 1 and 2). NMT2 overexpression in CD8+ T cell is statistically significant (Table 1). However, NMT2 from in CD4+ T cells shows a wide range of expression in both control and CRC groups (Figure 1). The highest H score from control group is from patient CS2 (H score = 40), and the lowest expression in CRC group is from ID024 (H score = 30).

### Patient Samples

Patient samples were obtained from Health Sciences Centre, Winnipeg, Manitoba after requisite ethics approval from University of Winnipeg, University of Manitoba, Health Sciences Centre and patient consent. In total 4 controls and 4 CRC patients, and 3 patients with unusual NMT2 expression were examined. All patients have undergone colonoscopy. Patients diagnosed with CRC were placed in Group 1. Control subjects with no evidence of CRC, polyps or family history were placed in the Group 2. Patients with Inflammatory Bowel Disease (IBD), diverticula, or a family history of CRC who did not show symptoms of CRC were placed in Group 3. Polyclonal human anti-NMT1 and human anti-NMT2 antibodies were procured from Sigma Canada. These antibodies were prestige series validated for IHC. They are specific for NMT1 or NMT2.

### PBMC separation

PBMCs were isolated from samples from CRC patients or control subjects. Blood samples were carefully transferred into a 50mL centrifuge tube and diluted with RPMI 1640 media in a 1:1 ratio. RPMI 1640 that was supplemented with 1% sodium-pyruvate, 1% L-glutamine was used for dilution of blood samples. 15 mL centrifuge tubes were used that were filled with 4mL Ficoll. Blood was slowly poured onto the Ficoll so as not to disturb the Ficoll surface. These were centrifuged at 800g for 30min at room temperature. Peripheral Blood Mononuclear Cells (PBMC) float to the top of Ficoll column while red blood cells settle below it. Blood plasma settles above the PBMC layer. Plasma was removed using pipette. The PBMC layer was gently pipetted out into a 15mL centrifuge tube and the rest was discarded. The isolated PBMC were washed twice by mixing cells in RPMI 1640 and centrifugation at 320g for 10 min. Cells were then pelleted down and re-suspended in 3ml of RPMI for counting. Trypan blue exclusion method was used to count viable cells. Based on the cell count the samples were diluted accordingly to allow for T cell isolation and cytospin fixing.

### CD8+ and CD4+ T cell Isolation

T cell isolation was performed through negative selection by use of magnetic beads (Dynabeads from Invitrogen). The beads are coated with anti-mouse antibodies. These antibodies are present on PBMC other than T cell subpopulation of interest. The PBMC were separated into two aliquots in 1:2 ratio for T cell separation. 70% of PBMC are T cells, and there are twice as many CD4+ T cells as CD8+ T cells. The 1:2 separation allows equal amounts of both types of cells to be recovered. These aliquots were incubated on ice for 30 minutes with antibodies against cells that needed to be removed. Then cells were washed twice to remove excess antibodies. The cells were diluted to a concentration of at least 10⁷ cells/ml for incubation with magnetic beads. Each cell needs to be bound to 4 beads for optimum separation and appropriate quantities were used. The cells were incubated with magnetic beads for 30min on ice. After incubation with beads the cells were diluted to a volume of 5ml. To separate the cells of interest the PBMC-bead aliquot was exposed to a magnet for 2 minutes. Magnetic beads attached to unwanted cells coagulate around the magnets and T cells of were poured out with the supernatant. Isolated T cells were counted, diluted and prepared for fixation using procedure mentioned for PBMC isolation. Alternatively, CD8+ T cells were positively selected by using CD8 antibody and CD4+ cells were positively selected by CD4+ antibody conjugated to magnetic beads. After positive selection of CD4+ T cells or CD8+ T cells, magnetic beads were removed by detach-a-bead reagent.

### Cytospin

Cytospin allows cells to be fixed onto slides using centrifugation. Samples containing at least 100,000 live cells suspended in 200µl of RPMI 1640 were mounted onto slides using cytospin apparatus (cytospin chamber and clip). When put through a centrifugation cycle, the suspension fluid disperses into the filter paper on the base of the funnel and centrifugational force fixes cells onto slides.

### IHC analysis using the Enhanced Polymer One Step (EPOS) method

The primary antibody (rabbit anti-human) binds to antigen of interest. Multiple secondary anti-bodies (goat anti-rabbit) are bound to a dextran polymer backbone and localises around the primary antibody. Multiple Horse Radish Peroxidase enzymes are also couples to the dextran backbone. When the chromagen 3,3'-Diaminobenzidine (DAB) is introduced it is oxidised in the vicinity of the target protein to produce a dark brown stain (Dako, 2011).

### IHC analysis

Standard IHC techniques were used to localize antigens of interest i.e. NMT1 and NMT2 using polyclonal antibodies specific for NMT1 or NMT2. IHC analysis was performed on, whole blood smears, PBMC, CD4+ T cells and CD8+ T cells using automated Ventanna system at the CancerCare Manitoba, Winnipeg. The primary antibodies used in this study are: NMT1 and NMT2 (polyclonal rabbit anti-human, 1:50 dilution). HRP conjugated secondary antibodies were used that reacted with chromagen 3,3'-Diaminobenzidine (DAB) to produce a brown stain. The intensity of the DAB stain is a measure of the amount of protein present.

### Quantifying IHC results

Slides were quantified based on their IHC-score. An IHC-score (H score) is product of staining intensity (on a scale of 0 to 3, a 3 being the highest) and percentage of cells that take up the staining. For example, a sample where 90% of the cells stain positive and the stain intensity is 3, the H score is 3*90 = 270. H scores range from 0 to 300. Statistical analysis was done using a two-tailed T-test. A T-test is used to establish whether two sets of data being compared are part of a larger parent data set. If two sets of data belong to the same normally distributed data, then the observed differences could be due to chance. Here, a T-test is used to assess whether a difference between the H scores from control and CRC groups are a coincidence.

The scope of the claims should not be limited by the preferred embodiments set forth in the examples, but should be given the broadest interpretation consistent with the description as a whole.

### REFERENCES

Aitken, A., Cohen, P. & Santikarn, S., 1982. Identification of the NH2-terminal blocking group of calcineurin B as myristic acid. FEBS Letter, pp. 314-8.
   Anon., 2006. Population screening for colorectal cancer. Drug ThurBull, Volume 44, pp. 65-68.
Baxter, N. & Rabeneck, L., 2009. Is the effectiveness of colonoscopy "good enough" for population based screening?. J Natl Cancer Inst, pp. 70-1.
   Boutin, J., 1997. Myristoylation. Cell Signal, pp. 15-35.
Canadian Cancer Society, 2010. Canadian Cancer Society's Steering Committee. Toronto, Cancer Statistics.
CancerCare Manitoba, 2007. Cancer in Manitoba: 2004 Annual Statistics Report, Winnipeg: s.n.
Carr, S. et al., 1982. n-Tetradecnoyl is the NH2-terminal blocking group of the catalytic subunit of cyclic AMP-dependent protein kinase from bovine cardiac muscle. USA, s.n., pp. 6128-31.
Deckers, P., Davis, R., Parker, G. & Mannick, J., 1973. The effect of tumour size on concomitant tumour immunity. CancerRes, pp. 33-9.
Duffy, M., Dalen, A. & Haglund, C., 2003. Clinical utility of biochemical markers in colorectal cancer: European Group on Tumour Marker guidelines. Eur J Cancer, pp. 718-27.
Farazai, T., Waksman, G. & Gordon, J., 2001. The biology and enzymology of protein N-myristoylation. J Biol Chem, pp. 39501-4.
Furuishi, K. et al., 1997. Blockage of N-myristoylation of HIV-1 gag induces the production of impotent progeny virus,. Biochem. Biophys. Res. Commun., pp. 504-511.
Giang, D. & Cravatt, B., 1998. A second mammalian N-myristoyltransferase. J Biol Chem, pp. 6595-8.
Irving, K., 2010. Tumour immunology: Tumours support the T cell response. Nature Reviews Immunology, p. 617.
King, M. & Sharma, R., 1991. NMT assay using phosphocellulose paper binding. Anal Biochem, pp. 149-53.
Kumar, A. & Singh, S., 1995. Effect of cisplatin administration on the proliferation and differentiaion of bone marrow cells of tumour-bearing mice. Immunol Cell biol, pp. 220-5.
Magnuson, B., Raju, R., Moyana, T. & Sharma, R., 1995. Increased N-myristoyltransferase activity observed in rat and human colonic tumours. J Natl Cancer, pp. 1630-5.
Marc, D. et al., 1989. Role of myristoylation of poliovirus capsid protein VP4 as determined by site-directed mutagenesis of its N-terminal sequence. Embo K, pp. 2661-8.
Martin, D., Beauchamp, E. & Berthiaume, L., 2011. Post-translational myristoylation: Fat matters in cellular life and death. Biochimie, pp. 18-31.
Maurer-Stroh, S. & Eisenhaber, F., 2004. Myristoylation of viral and bacterial proteins. Trends Microbiol, pp. 178-185.
Moayyedi, P., 2007. Colorectal cancer screening lacks evidence of benefit. Cleve Clin J Med, pp. 549-550.
Nicholson, F., Barro, J. & Atkin, W., 2005. Population screening for colorectal cancer. Aliment Pharmacol Ther, pp. 1069-77.
Orkin, S. & Zon, L., 2008. Hematopoiesis: An Evolving Paradigm for Stem Cell Biology. Cell, pp. 631-644.
Ouyang, D., Chen, j., Getzenberg, R. & Schoen, R., 2005. Noninvasive testign for colorectal cancer: a review. Am J Gastroenterology, pp. 1393-403.
Rajala, R. et al., 2000. Increased expression of NMT in gallbladder carcinomas. Cancer, pp. 1992-9.
Rein, A. et al., 1986. Myristoylation site in Pr65gag is essential for virus particle formation by moloney nurine leukemia virus.. USA, s.n., pp. 7246-50.
Resh, M., 1999. Fatty acylation of proteins - new insights into membrane targetign of myristoylated and palmitoylated proteins. Biochim Biophys Acta, Volume 1451, pp. 1-16.
Resh, M., 2004. A myristoyl switch regulates the membrane binding of HIV-1 Gag.. s.l., Proc Natl Acad Sci, pp. 417-8.
Schultz, A. et al., 1987. Hydroxylanime-stable covalent linkage of myristic acid in G0 alpha, a guanine nucleotide-binding protein of bovine brain.. Biochem Biophys Res Commun, pp. 1234-9.
Schultz, A. et al., 1985. Amino terminal myristoylation of the protein p60src, a retroviral transforming protein. Science, pp. 427-9.
Screening, C. C., 2001. Reccomendation statement from the Canadian Task force on Preventive Health Care. CMAJ, Issue 165, pp. 206-208.
Seaton, K. & Smith, C., 2008. N-Myristoyltransferase isozymes exhibit differential specificity for human immunodeficiency virus type 1 Gag and Nef. J. Gen. Virol., pp. 288-296.
Selvakumar, P., Smith-Windsor, E., Bonham, K. & Sharma, R., 2006. N-myristoyltransferase 2 expression in human colon cancer: cross-talk between the caplain and caspase system. FEBS Letter, pp. 2021-6.
Shrivastav, A., Varma, S. & Lawman, Z., 2008. Requirement of NMT1 in the development of monocytic lineasge. J Immunol, pp. 1019-28.
Shrivastav, A. et al., 2007. N-myristoylatransferase: a potential novel diagnostic marker for colon cancer.. J Transl Med, p. 58.
Shrivastav A, Suri SS, Mohr R, Janardhan KS, Sharma RK, Singh B. Expression and activity of N-myristoyltransferase in lung inflammation of cattle and its role in neutrophil apoptosis. Vet Res. 2010;41:9.
Singh, H. et al., 2010. The reduction in colorectal cancer mortality after colonoscopy varies by site of cancer. Gastroenterology.
Singh, H. et al., 2009. Predictors of colorectal cancer after negative colonoscopy: a population-based study. Am J Gastroenterology, pp. 663-73.
Towler, D., Adams, S. & Eubanks, S., 1987. Purification and myristoylation of yeast myristoyl CoA: protein N-myristoyltransferase. USA, s.n., pp. 2708-12.
Vilar, G. et al., 2006. Posttranslational myristoylation of caspase-activated p21-activated protein kinase 2 potentiates late apoptotic events. USA, s.n., pp. 6542-7.
Winawer, S. et al., 2003. Colorectal cancer screening and surveilance: clinical guidelines and rationale-Update based on new evidence. Gastroenterology, Volume 124, pp. 544-560.
Yang, S. et al., 2005. N-myristoyltransferase 1 is essential in early mouse development. J Biol chem, pp. 18990-5.
Zha, J. et al., 2000. Posttranslational N-myristoylation of BID as a molecular switch for targeting mitochondria and apoptosis. Science, pp. 1761-5.

**Table 1 Comparison of NMT1 and NMT2 expression groups in CD4+ T cells and CD8+ T cells in patient and control. NMT2 is found to be overexpressed in CD8+ T cells by two tailed T-test (Pα = 0.05)**

| **NMT *vs* T cell** | **P-value (Pα=0.05)** | **Significant Difference** |
|---|---|---|
| **NMT1** | | |
| **CD4** | 0.091 | No |
| **CD8** | 0.243 | No |

| **NMT2** | | |
|---|---|---|
| **CD4** | 0.082 | No |
| **CD8*** | 0.026 | Yes |

| | | |
|---|---|---|
| *NMT2 is found to be overexpressed in CD8+ T cells from CRC patients | | |

**Table 2 NMT1 Expression profile of patients in CD4+ T cells and CD8+ T cells (n=11)**

| **Patient ID** | **CD4** | | | **CD8** | | |
|---|---|---|---|---|---|---|
| | Intensity | Penetrance | H-Score | Intensity | Penetrance | H-Score |
| **Group 1 (Control)** | | | | | | |
| **CS1** | 01 | 05 | 05 | 01 | 05 | 40 |
| **CS2** | 01 | 05 | 05 | 01 | 05 | 05 |
| **ID012** | 01 | 05 | 05 | 01 | 05 | 05 |
| **ID017** | 01 | 05 | 05 | 01 | 20 | 20 |
| | | | | | | |

| **Group 2 (CRC Patients)** | | | | | | |
|---|---|---|---|---|---|---|
| **ID015** | 01 | 20 | 20 | 01 | 30 | 30 |
| **ID018** | 01 | 20 | 20 | 01 | 90 | 90 |
| **ID023** | 01 | 05 | 05 | 01 | 10 | 10 |
| **ID024** | 01 | 05 | 05 | 01 | 05 | 05 |
| | | | | | | |

| **Group 3 (No CRC with high NMT)** | | | | | | |
|---|---|---|---|---|---|---|
| **ID002** | 02 | 40 | 80 | 02 | 30 | 60 |
| **ID008** | 01 | 30 | 30 | 01 | 30 | 30 |
| **ID013** | 01 | 70 | 70 | 01 | 70 | 70 |

**Table 3 NMT2 Expression profile of patients in CD4+ T cells and CD8+ T cells (n=11)**

| **Patient ID** | **CD4** | | | **CD8** | | |
|---|---|---|---|---|---|---|
| | Intensity | Penetrance | H-Score | Intensity | Penetrance | H-Score |
| **Group 1 (Controls)** | | | | | | |
| **CS1** | 01 | 05 | 05 | 01 | 05 | 05 |
| **CS2** | 01 | 40 | 40 | 01 | 40 | 40 |
| **ID012** | 01 | 05 | 05 | 01 | 05 | 05 |
| **ID017** | 01 | 05 | 05 | 01 | 20 | 20 |
| | | | | | | |

| **Group 2 (CRC Patients)** | | | | | | |
|---|---|---|---|---|---|---|
| **ID015** | 02 | 30 | 60 | 03 | 40 | 120 |
| **ID018** | 03 | 90 | 120 | 03 | 90 | 180 |
| **ID023** | 01 | 30 | 30 | 03 | 50 | 150 |
| **ID024** | 03 | 30 | 90 | 03 | 20 | 60 |
| | | | | | | |

| **Group 3 (No CRC with high NMT)** | | | | | | |
|---|---|---|---|---|---|---|
| **ID002** | 01 | 20 | 20 | 03 | 90 | 120 |
| **ID008** | 02 | 90 | 180 | 02 | 80 | 160 |
| **1D013** | 02 | 90 | 180 | 03 | 90 | 180 |

**Table 4: Medical backgrounds of participants (n=11)**

| **Patient ID** | **Medical History** |
|---|---|
| **Group 1 (Controls)** | |
| **CS1** | No known instances of cancer |
| **CS2** | Breast Cancer |
| **ID012** | Breast Cancer |
| **ID017** | N/A |
| | |

| **Group 2 (CRC Patients)** | |
|---|---|
| **ID015** | Sigmoid cancer Stage 4. No polyps. |
| **ID018** | CRC Stage 3c. Has polyps |
| **ID023** | CRC Stage 4. Family history. |
| **ID024** | Rectal Adenocarcinoma. Initial. |
| | |

| **Group 3 (No CRC with high NMT)** | |
|---|---|
| **ID002** | Mother has breast cancer. CRC resected in 2010. |
| **ID008** | No cancer. Family history of CRC. Has polyps. |
| **ID013** | No cancer. Has diverticulitis in sigmoidal colon |

## Claims

1. A method of identifying a candidate for further colorectal cancer (CRC) screening comprising:
measuring N-myristoylatransferase 2 (NMT2) levels in a sample from a patient at risk of developing colorectal cancer, suspected of having colorectal cancer or having colorectal cancer, wherein NMT2 levels above a threshold level indicates that the patient is a candidate for further CRC screening, wherein the sample is selected from the group consisting of: whole blood, peripheral blood monocyte cells, T-cells and/or CD8+ cells.

2. The method according to claim 1, wherein the further colorectal cancer screening is selected from the group consisting of: sigmoidoscopy and colonoscopy.

3. The method according to claim 1, wherein the NMT2 levels are measured using a primer comprising nucleic acid sequence unique to NMT2.

4. The method according to claim 1, wherein the NMT2 levels are measure using a probe comprising nucleic acid sequence unique to NMT2.

5. The method according to claim 1, wherein the NMT2 levels are measured using an antibody specific for NMT2.

6. The method according to claim 1, wherein the patient is a human.

7. The method according to claim 6, wherein the individual has a familial history of colorectal cancer or is older than 55.

## Patentansprüche

1. Verfahren zur Identifizierung eines Kandidaten für ein weiteres Darmkrebs-(CRC)-Screening, umfassend:
Messen von N-Myristoyltransferase-2-(NMT2)-Spiegeln in einer Probe von einem Patienten, der ein Risiko hat, Darmkrebs zu entwickeln, bei dem vermutet wird, dass er Darmkrebs hat, oder der Darmkrebs hat, wobei ein NMT2-Spiegel über einem Schwellenwert darauf hindeuten, dass der Patient ein Kandidat für weiteres CRC-Screening ist, wobei die Probe ausgewählt ist aus der Gruppe bestehend aus Vollblut, periphere Blutmonozytenzellen, T-Zellen und/oder CD8+-Zellen.

2. Verfahren nach Anspruch 1, wobei das weitere Darmkrebs-Screening ausgewählt ist aus der Gruppe bestehend aus: Sigmoidoskopie und Koloskopie.

3. Verfahren nach Anspruch 1, wobei die NMT2-Spiegel unter Verwendung eines Primers gemessen werden, der eine Nukleinsäuresequenz umfasst, die für NMT2 einzigartig ist.

4. Verfahren nach Anspruch 1, wobei die NMT2-Spiegel unter Verwendung einer Probe gemessen werden, die eine Nukleinsäuresequenz aufweist, die für NMT2 einzigartig ist.

5. Verfahren nach Anspruch 1, wobei die NMT2-Spiegel unter Verwendung eines für NMT2 spezifischen Antikörpers gemessen werden.

6. Verfahren nach Anspruch 1, wobei der Patient ein Mensch ist.

7. Verfahren nach Anspruch 6, wobei das Individuum eine Familiengeschichte von Darmkrebs aufweist oder älter als 55 ist.

## Revendications

1. Procédé d'identification d'un candidat à un dépistage approfondi du cancer colorectal (CRC), comprenant :
la mesure de taux de N-myristoyltransférase 2 (NMT2) dans un échantillon d'un patient à risque de développement d'un cancer colorectal, supposé avoir un cancer colorectal ou ayant un cancer colorectal, un taux de NMT2 supérieur à un niveau seuil indiquant que le patient est candidat pour un dépistage approfondi du cancer colorectal, l'échantillon étant sélectionné dans le groupe comprenant : sang total, cellules monocytes du sang périphérique, cellules T et/ou cellules CD8+.

2. Procédé selon la revendication 1, où le dépistage approfondi du cancer colorectal est sélectionné dans le groupe comprenant : sigmoïdoscopie et colonoscopie.

3. Procédé selon la revendication 1, où les taux de NMT2 sont mesurés au moyen d'une séquence d'acide nucléique comprenant une amorce, propre à la NMT2.

4. Procédé selon la revendication 1, où les taux de NMT2 sont mesurés au moyen d'une séquence d'acide nucléique comprenant une sonde, propre à la NMT2.

5. Procédé selon la revendication 1, où les taux de NMT2 sont mesurés au moyen d'un anticorps spécifique à la NMT2.

6. Procédé selon la revendication 1, où le patient est humain.

7. Procédé selon la revendication 6, où l'individu a des antécédents familiaux de cancer colorectal ou a dépassé les 55 ans.
